# EUROPEAN PATENT APPLICATION

(11) **EP 4 299 728 A1**
(43) Date of publication of application: **03.01.2024**
(21) Application number: 22305938.7
(22) Date of filing: 28.06.2022
(51) Int. Cl.: C12N 7/00

(54) **RECOMBINANT SPRIVIVIRUSES, USES THEREOF AND METHODS FOR PRODUCING THE SAME**

(71) Applicant: Institut national de recherche pour l'agriculture, l'alimentation et l'environnement, 75007 Paris (FR); UNIVERSITE DE VERSAILLES-SAINT QUENTIN EN YVELINES, 78035 Versailles cedex (FR); Universidade de Santiago de Compostela, 15782 Santiago de Compostela, A Coruña (ES)
(72) Inventor: BIACCHESI, Stéphane, 94170 LE PERREUX SUR MARNE (FR); MEROUR, Emilie, 91360 EPINAY SUR ORGE (FR); MILLET, Jean, 78350 JOUY-EN-JOSAS (FR); SOUTO PEREIRA, Sandra, 15706 SAINT JACQUES DE COMPOSTELLE (ES)
(74) Representative: Gevers & Orès

(57) **Abstract**

The present invention relates to recombinant spriviviruses, in particular a recombinant spring viraemia of carp virus, uses thereof and methods for producing the same.

## Description

The present invention relates to recombinant spriviviruses, in particular a recombinant spring viraemia of carp virus, uses thereof and methods for producing the same.

Spring viraemia of carp virus (SVCV) belongs to the genus *Sprivivirus.* It has a broad host spectrum (carp, pike, sturgeon, goldfish, etc.) and is responsible for an infectious disease which generally affects juvenile fishes and spreads rapidly in farms, with a mortality rate of up to 90%. The presence of the virus is reported in ponds throughout France, as well as in the main producing countries of cyprinids (Asia, Eastern Europe, etc.). Carp farming ranks second in fish production in France. Depending on the species, the production systems are semi-intensive or intensive fish farms, intended either for food consumption (production exceeding 28 million tons per year; Food and Agriculture Organization 2015) or for the production of ornamental varieties, such as koi carp whose unit prices may exceed €100,000. In the absence of treatments or vaccine against SVCV, the search for prophylactic or therapeutic solutions is thus relevant, particularly to identify solutions compatible with mass vaccination needs (i.e. bathing rather than injection of vaccines).

In addition to this aspect, SVCV is used as a model virus for the study of host-pathogen relationships of several hemorrhagic viruses (Ebola, dengue fever, Lassa, etc.), for which it remains difficult to develop *in vivo* models due to limits of the murine model or complexity of the primate model. The zebrafish, which is widely used as a biomedical model because of its small size and its properties of optical transparency particularly appreciated for the screening of therapeutic molecules, can be experimentally infected with SVCV.

The genome of SVCV consists of a single molecule of negative-sense, single-stranded RNA of approximately 11 kb. The genomic RNA contains five genes in the order 3'-N-P-M-G-L-5' encoding a nucleoprotein (N), a phosphoprotein (P), a matrix protein (M), a glycoprotein (G) and a RNA-dependent RNA polymerase (L) respectively. However, although the genomic structure of SVCV has been characterized, there are still no tools available allowing its genetic manipulation and the production of recombinant viral particles.

Thus, there remains a genuine need for an effective system allowing the genetic manipulation of spriviviruses, in particular SVCV.

The present invention is believed to meet such need by providing a reverse genetics system for spriviviruses.

The recombinant spriviviruses were obtained for the first time by co-transfecting hamster cell lines with an antigenomic cDNA, i.e. a copy of the viral genome in positive polarity, along with some expression plasmids encoding the viral proteins N, P and L, in order to form a viral replication complex. After transfection, the recombinant viruses were amplified on fish cells. The success of this approach relies on some permissive cells (i.e. some cells that support virus replication) and a compromise between the optimal culture temperature for hamster cells, which is about 37°C, and that of SVCV replication, which is about 25°C. This reverse genetics system thus provides a very powerful tool that enables directed manipulation of the genome of this virus, e.g. introducing targeted mutations, deleting a viral gene, adding additional genes or changing the order of genes.

In addition, the Inventors developed several genetic tools allowing the modification of the viral genome and the use of the recombinant spriviviruses as gene vectors or vaccine platforms. These tools allow either the cytoplasmic expression of heterologous genes or their expression on the membrane of the infected cells and, thus, their incorporation into the viral particles.

In an aspect, the present invention thus concerns a recombinant virus of the genus *Sprivivirus* comprising in its genome at least one exogenous genetic sequence.

In the present invention, the expression "recombinant virus" designates a "viral particle" or "virion", i.e. a particle formed by a lipid envelope, a linear RNA genome and some viral proteins, which further comprises in its genome an exogenous genetic sequence, i.e. a coding or non-coding nucleotide sequence that is not naturally present in the genome of the virus.

In the present invention, the expression "exogenous genetic sequence" designates any nucleotide sequence that is not naturally present in the genome of the virus. In other words, it designates any nucleic acid sequence that has been introduced or modified in the viral genome by genetic manipulation, e.g. a sequence introduced in the viral genome in addition to the endogenous viral genes, a sequence introduced in the viral genome in substitution to an endogenous viral gene, or a modified viral genome or segment thereof wherein the order of the genes has been rearranged.

In an embodiment, the length of the exogenous genetic sequence is at least 10 bases, preferably at least 100 bases, more preferably at least 500 bases, even more preferably at least 1 000 bases (1Kb).

In an embodiment, the length of the exogenous genetic sequence is at least 10, 50, 100, 200, 500, 1 000, 2 000, 3 000, 4 000 or 5 000 bases.

In an embodiment, the length of the exogenous genetic sequence is from 100 to 5 000 bases, preferably from 500 to 5 000 bases, more preferably from 1 000 to 5 000 bases.

In an embodiment, the invention relates to a recombinant virus as defined above, which comprises more than one exogenous sequence, preferably two exogenous sequences.

In an embodiment, the present invention relates to a recombinant virus of the genus Sprivivirus comprising in its genome at least one exogenous genetic sequence encoding a heterologous gene, in addition to the endogenous viral genes encoding nucleoprotein (N), phosphoprotein (P), matrix protein (M), glycoprotein (G) and polymerase (L).

In an embodiment, the present invention relates to a recombinant virus of the genus Sprivivirus comprising in its genome at least one exogenous genetic sequence encoding a heterologous gene, in substitution of an endogenous viral gene encoding nucleoprotein (N), phosphoprotein (P), matrix protein (M) or glycoprotein (G).

According to the present invention, a "heterologous gene" encodes any peptide or protein different from the N, P, M, G and L proteins of spriviviruses. For example, a heterologous gene of interest can be an antigen or a fluorescent or bioluminescent reporter protein.

In an embodiment, the invention relates to a recombinant virus as defined above, wherein the exogenous sequence encodes more than one heterologous gene, preferably two heterologous genes.

In an embodiment, the invention relates to the recombinant virus as defined above, wherein the exogenous genetic sequence comprises or consists of an expression cassette encoding a peptide or protein of interest.

In the present invention, the term "expression cassette" designates any nucleic acid molecule comprising at least one gene and the sequences controlling its expression.

Basically, an expression cassette comprises a promoter, a gene of interest (open reading frame, ORF) and a terminator. For some applications, recombinant viruses comprising several expression cassettes can be of particular interest when it is desired to simultaneously express several polypeptides, e.g. two subunits of the same enzyme, or an inactive precursor of a protein and a second protein capable of converting said precursor into the active form of the protein.

In order to facilitate the expression of proteins of interest by spriviviruses, the Inventors have identified the initiation (Gene Start; GS) and termination/polyadenylation (Gene End; GE) signals recognized by the viral RNA-dependent RNA polymerase. A heterologous gene flanked by these two specific sequences can thus be inserted into the cDNA of the virus and be expressed efficiently in the host cell during viral replication.

In an embodiment, the invention relates to a recombinant virus as defined above, wherein the expression cassette comprises, from 3' end to 5' end:
- a transcription initiation Gene Start (GS) sequence chosen from the group consisting in SEQ ID NO: 1 (UUGUCUCUAGUAC) and SEQ ID NO: 2 (UUGUCUGUAGUAC),
- a sequence encoding the protein of interest, and
- a transcriptional termination/polyadenylation Gene End (GE) sequence consisting in SEQ ID NO: 3 (AUACUUUUUUU),
said expression cassette being inserted in a non-coding region of the genome.

In an embodiment, the invention relates to the recombinant virus as defined above, wherein the expression cassette comprises, from 3' end to 5' end: a transcription initiation Gene Start (GS) sequence consisting in SEQ ID NO: 1 (UUGUCUCUAGUAC), a sequence encoding the protein of interest, and a transcriptional termination/polyadenylation Gene End (GE) sequence consisting in SEQ ID NO: 3 (AUACUUUUUUU), said expression cassette being inserted in a non-coding region of the genome.

In an embodiment, the invention relates to the recombinant virus as defined above, wherein the expression cassette comprises, from 3' end to 5' end: a transcription initiation Gene Start (GS) sequence chosen from the group consisting in SEQ ID NO: 2 (UUGUCUGUAGUAC), a sequence encoding the protein of interest, and a transcriptional termination/polyadenylation Gene End (GE) sequence consisting in SEQ ID NO: 3 (AUACUUUUUUU), said expression cassette being inserted in non-coding region of the genome.

The sequences of the exogenous sequences or of their genetic elements are given as they are in the single molecule of negative-sense, single-stranded RNA contained in the viral particle.

The non-coding region can be the leader region, an intergenic region or the trailer region of the viral genome, in particular an intergenic region of the viral genome.

Alternatively, the Inventors have shown that an additional gene can be expressed in spriviviruses by using ribosomal skipping, in particular by using 2A-like sequences.

In an embodiment, the invention relates to the recombinant virus as defined above, wherein the exogenous genetic sequence comprises:
- a 2A-like sequence, which encodes a peptide that induces ribosomal skipping during translation, and
- a sequence encoding a protein of interest,
said exogenous genetic sequence being fused to the translated region of an endogenous viral gene encoding N, P, M, G or L, and the 2A-like sequence being located between the sequence encoding the protein of interest and the translated region of the endogenous viral gene encoding N, P, M, G or L.

In the present invention, the expression "2A-like sequence" designates a genetic sequence coding an oligopeptide sequence that mediates a ribosome skipping effect, producing an apparent co-translational cleavage of the polypeptide chain. This oligopeptide sequence generally has a core sequence motif of DXEXNPGP (SEQ ID NO: 4), wherein X is any amino acid.

In an embodiment, the 2A-like sequence encodes a peptide selected from the group consisting in P2A (ATNFSLLKQAGDVEENPGP, SEQ ID NO: 5), T2A (EGRGSLLTCGDVEENPGP, SEQ ID NO: 6), E2A (QCTNYALLKLAGDVESNPGP, SEQ ID NO: 7) and F2A (VKQTLNFDLLKLAGDVESNPGP, SEQ ID NO: 8), preferably P2A (SEQ ID NO: 5).

In a preferred embodiment, a sequence coding "GSG" residues is added to the 5' end of the 2A-like sequence to improve ribosomal skipping efficiency.

Alternatively, an additional gene can also be expressed in spriviviruses by using an exogenous genetic sequence which comprises an IRES (*Internal ribosome entry site*) sequence allowing translation initiation in a cap-independent manner and a gene of interest.

The present invention also renders possible to stably attenuate spriviviruses. Indeed, the expression of the genes follows a decreasing gradient along the viral genome according to the order 3'-N-P-M-G-L-5'. The first gene N is the most highly expressed and the last gene L is expressed the lowest. The addition of one or more genes in the genome has the effect of reducing the expression of the gene located downstream, which thus leads to the attenuation of the virus.

Thus, the invention also relates to a recombinant virus as defined above, wherein at least one exogenous genetic sequence is inserted in the leader/trailer regions or between two endogenous viral genes encoding nucleoprotein (N), phosphoprotein (P), matrix protein (M), glycoprotein (G) and polymerase (L).

In an embodiment, the invention relates to a recombinant virus as defined above, wherein at least one exogenous genetic sequence is inserted between M and G or G and L genes.

In an embodiment, the invention relates to a recombinant virus as defined above, wherein one exogenous genetic sequence is inserted between M and G genes.

In an embodiment, the invention relates to a recombinant virus as defined above, wherein one exogenous genetic sequence is inserted between G and L genes.

Preferably, the exogenous genetic sequence is inserted in a non-coding region.

More preferably, the exogenous genetic sequence comprises an expression cassette and is inserted in a non-coding region.

An alternative or complementary approach to stably attenuating the viruses is to change the order of viral genes. The inversion of the N and P or P and M genes are two examples that lead to the stable attenuation of the recombinant virus.

In an embodiment, the invention relates to a recombinant virus as defined above, wherein the endogenous viral genes encoding nucleoprotein N, P, M, G and L are not in the following order 3' N-P-M-G-L 5' .

In an embodiment, the invention relates to a recombinant virus as defined above, wherein the endogenous viral genes encoding nucleoprotein N, P, M, G and L are in the following orders 3' P-N-M-G-L 5' or 3' N-M-P-G-L 5'.

The recombinant virus according to the invention can also be used as a vector of antigens in order to induce an immune response against said antigens. To improve the efficiency of an antigenic response directed against heterologous antigens expressed by the virus, it is desirable that the antigens be exposed on the surface of the infected fish cells, or even on the surface of the viral particles, in order to promote its presentation to the host immune system. To this end, it is possible to insert into the genome of the virus a gene encoding a chimeric polypeptide containing the sequence of an antigen of interest fused at its N-terminal end by the sequence of the signal peptide (SP) of the G protein of a sprivivirus/novirhabdovirus and at its C-terminus the sequence of the transmembrane domain (TM) of the G protein of the sprivivirus. The expression of the gene results in the expression of the mature form of its translation product on the surface of the infected fish cells and its incorporation into the viral envelope, without interfering with the assembly of the virion nor with its ability to infect and replicate in cultured cells.

Thus, in an embodiment, the invention also relates to a recombinant virus as defined above, wherein the recombinant virus expresses a protein of interest flanked:
- at its N-terminus by the signal peptide sequence of the G protein, and
- at its C-terminus by the sequence of the transmembrane domain of the G protein or by the sequence of the membrane-proximal stem region and the transmembrane domain of the G protein.

Preferably, the signal peptide sequence and/or the sequence of the membrane-proximal stem region and/or the sequence of the transmembrane domain are from the G protein of the SVCV.

In an embodiment, the signal peptide sequence comprises or consists of SEQ ID NO: 9 (MSIISYIAFLLLIDSNLGIPI).

In an embodiment, the sequence of the transmembrane domain comprises or consists of SEQ ID NO: 10 (FGMTLVALILIFLLIRCCVACTYLMKRSKRPATESHEMRSLV)

In an embodiment, the membrane-proximal stem region comprises or consists of SEQ ID NO: 11 (IREDDIFFDNTGENGNPVDAVVEWVSGWGTSLKF).

Spriviviruses form a monophyletic group based on well-supported Maximum Likelihood or Maximum Clade Credibility trees inferred from complete L sequences. Spiviviruses share the same temperature requirements, the same genomic organization, infect common species and have the same GS and GE signals. This genus includes two virus species: Carp sprivivirus which has a single member, spring viraemia of carp virus (SVCV); and Pike sprivivirus which includes three recognized viruses, pike fry rhabdovirus (PFRV), grass carp rhabdovirus (GCRV) and tench rhabdovirus (TRV).

In an embodiment, the recombinant virus is chosen from the group comprising Carp sprivivirus and Pike sprivivirus species.

In an embodiment, the recombinant virus is chosen from the group comprising SVCV, PFRV, GCRV and TRV, preferably SVCV.

In another aspect, the invention concerns a cDNA molecule of the antigenome of a recombinant virus as defined above.

Such a cDNA molecule can be transfected into hamster kidney cells expressing T7 RNA polymerase in order to obtain viral particles of recombinant spriviviruses.

In another aspect, the invention concerns a method for producing a recombinant virus as defined above, said method comprising a step of cultivating some hamster kidney cells expressing T7 RNA polymerase that have been transfected with a cDNA molecule of the antigenome of the recombinant virus and at least one helper plasmid encoding N, P and L.

In an embodiment, some hamster kidney cells expressing T7 RNA polymerase have been transfected with a cDNA molecule of the antigenome of the recombinant virus and three helper plasmids encoding N, P and L respectively.

In an embodiment, the hamster kidney cells are derived from BHK-21 cells, preferably the hamster kidney cells are BSR-T7/5 cells.

In an embodiment, the transfected cells are incubated at 37°C for 12 hours and, then, incubated at 25°C for 7 days.

In an embodiment, the method as defined above further comprises a step of putting the transfected cells or a culture supernatant of the transfected cells into contact with some fish cells, in order to amplify the recombinant virus on the fish cells.

In another aspect, the invention relates to a recombinant virus obtained by the method as defined above.

In another aspect, the invention also relates to a recombinant virus as defined above for use as a vaccine.

In an embodiment, the invention concerns a recombinant virus for its use as a vaccine as defined above, wherein the recombinant virus induces an immune response directed against an antigenic protein of interest expressed by said recombinant virus.

In an embodiment, the invention concerns a recombinant virus for its use as a vaccine as defined above, wherein said vaccine is selected from the group comprising an anti-viral vaccine, an antibacterial vaccine, an anti-fungal vaccine, an anti-parasite vaccine or an anti-tumor vaccine.

In an embodiment, the invention concerns a recombinant virus for its use as a vaccine as defined above, wherein said vaccine is intended to be administered to an animal, preferably a fish, a bird or a mammal, more preferably a fish.

In an embodiment, the invention concerns a recombinant virus for its use as a vaccine as defined above, wherein said vaccine is intended to be administered to a fish chosen from the group comprising common carp (*Cyprinus carpio carpio*), koi carp (*Cyprinus carpio koi*)*,* crucian carp (*Carassius carassius*), silver carp (*Hypophthalmichthys molitrix*)*,* bighead carp (*Aristichthys nobilis*)*,* grass carp (white amur) (*Ctenopharyngodon idella*), goldfish (*Carassius auratus*), orfe (*Leuciscus idus*), tench (*Tinca tinca*), bream (*Abramis brama*), European catfish or wels (*Silurus glanis*), pike (*Esox lucius*)*,* Nile tilapia (*Sarotherodon niloticus*)*,* Siberian sturgeon (*Acipenser baerii*)*,* Zebrafish (*Danio rerio*), Golden shiner (*Notemigonus crysoleucas*), Fathead minnow (*Pimephales promelas*), Caspian white fish (*Rutilus kutum*) and Roach (*Rutilus rutilus*)*.*

In another aspect, the invention relates to a non-therapeutic method for producing antibodies directed against an antigenic protein of interest, said method comprising the immunization of a non-human animal with a recombinant virus as defined above, said recombinant virus expressing the antigenic protein of interest. These antibodies can be obtained by recovery of the serum thereof (for the production of polyclonal antibodies) or of the lymphocytic cells thereof (for the production of monoclonal antibodies).

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

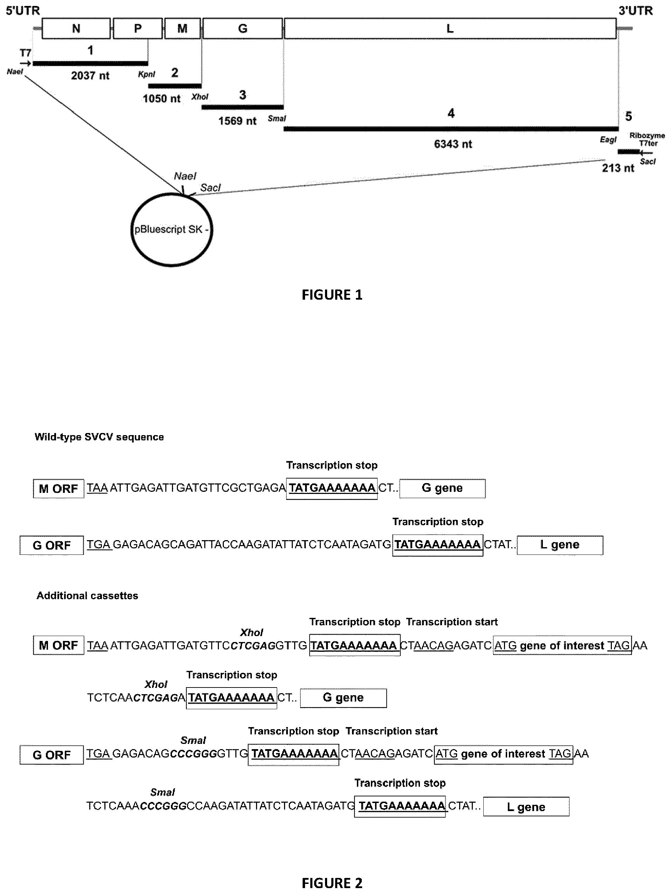

**Figure 1****.** Antigenomic SVCV infectious cDNA construct.
**Figure 2****.** Insertion of additional expression cassettes in SVCV genome.

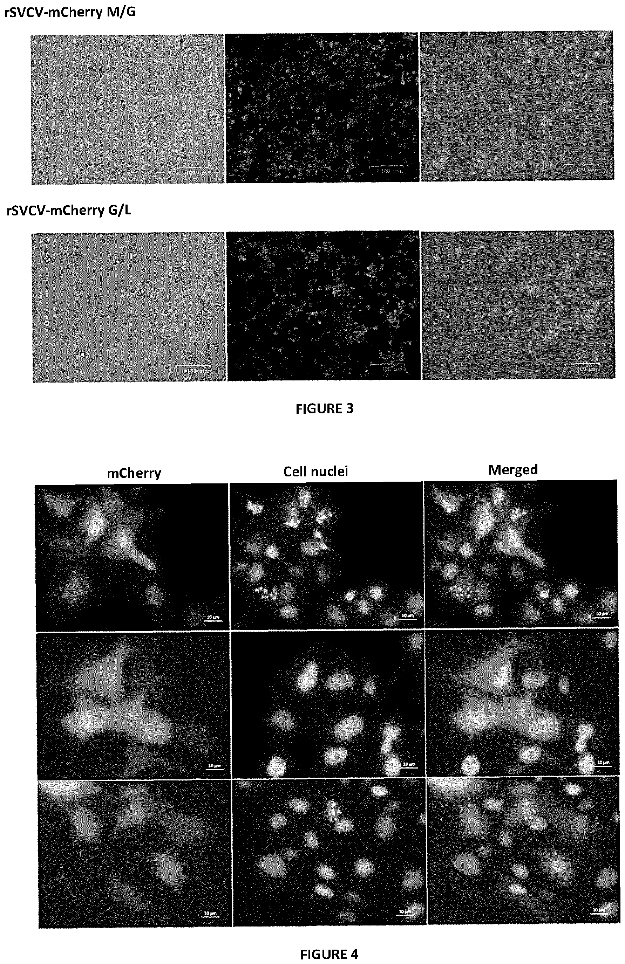

**Figure 3****.** Expression of mCherry fluorescent protein in live rSVCV-mCherry M/G or rSVCV-mCherry G/L infected EPC cells. The cells were incubated at 25 °C for 24 hours after infection. Bars, 100 µm.
**Figure 4****.** Expression of mCherry fluorescent protein in live rSVCV-mCherry M/G infected EPC cells. The cells were incubated at 25 °C for 24 hours after infection. Cell monolayers were then visualized with a UV-light microscope (Carl Zeiss) after nuclei staining with Hoechst solution in the cell culture medium. Magnification ×63. Bars, 10 µm.

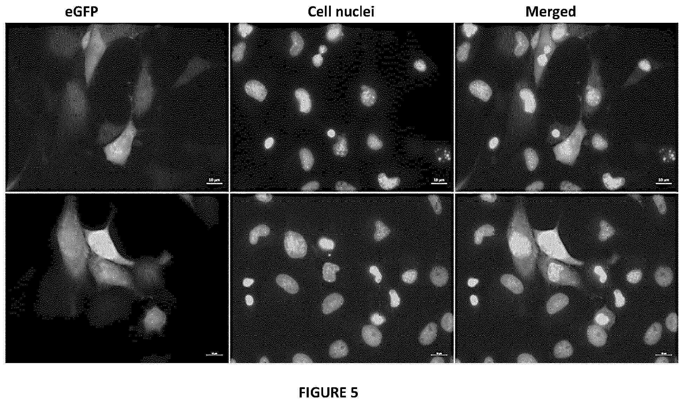

**Figure 5****.** Expression of eGFP fluorescent protein in live rSVCV-GFP infected EPC cells. The cells were incubated at 25 °C for 24 hours after infection. Cell monolayers were then visualized with a UV-light microscope (Carl Zeiss) after nuclei staining with Hoechst solution in the cell culture medium. Magnification ×63. Bars, 10 µm.

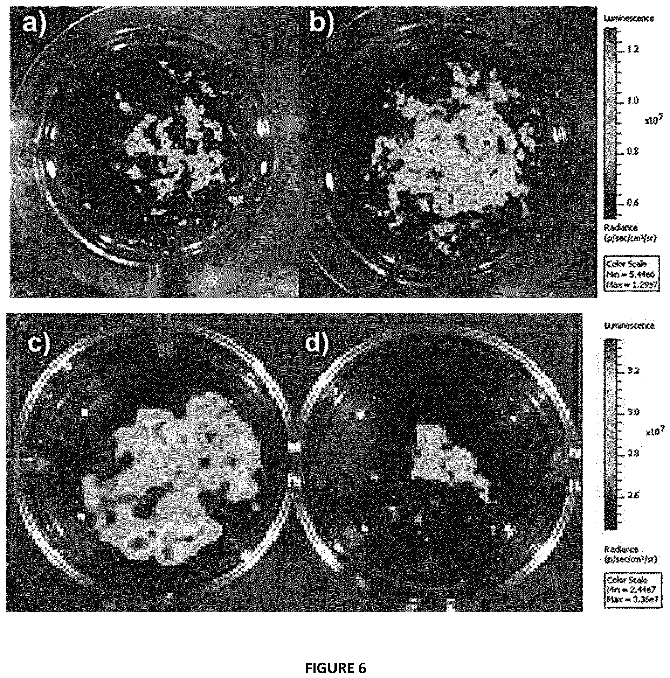

**Figure 6****.** Expression of firefly luciferase protein in live EPC cells infected with rSVCV-fLUC M/G (a and b) or rSVCV-akaLUC G/L (c and d). a) and b) Cells were infected at a MOI of 0.1. At 10 hours post-infection (hpi) (a) and 17 hpi (b) cells were washed three times with sterile Phosphate Buffer Saline (PBS) and the D-luciferin substrate (Caliper) was added at a concentration of 250 µM. The luminescence was measured using the IVIS Spectrum BL imaging system (Perkin Elmer) c) and d) Cells were infected at a MOI of 0.1 (c) or 0.01 (d). At 24 hours hpi, cells were washed three times with sterile PBS and the akalumine-HCI substrate (Sigma) was added at a concentration of 2.5 µM. The luminescence was measured using the IVIS Spectrum BL imaging system (Perkin Elmer).

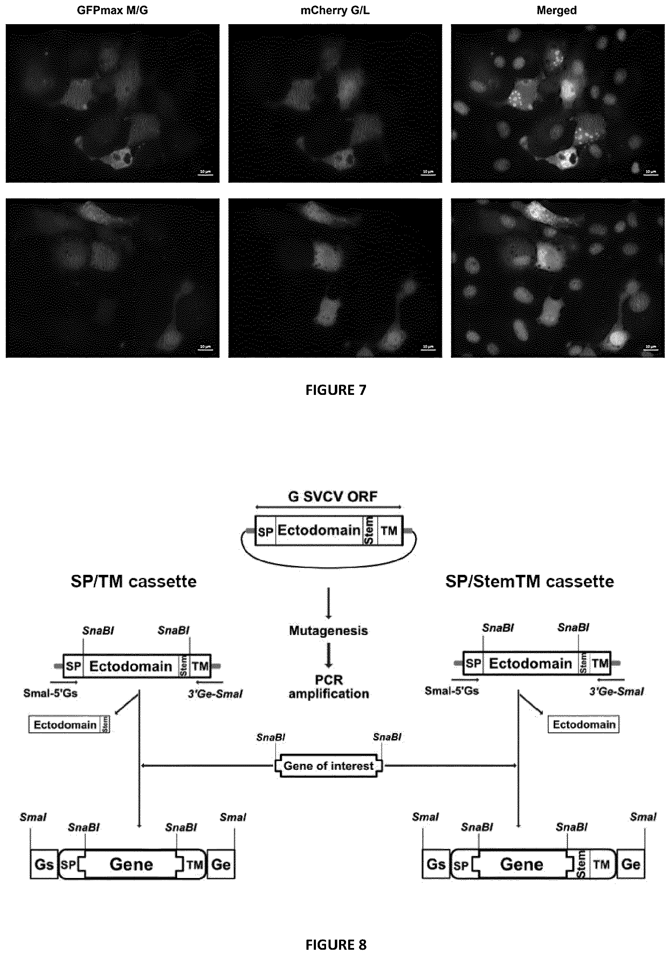

**Figure 7****.** Expression of GFPmax and mCherry fluorescent proteins in live rSVCV-GFPmax-mCherry infected EPC cells. The cells were incubated at 25 °C for 24 hours after infection. Cell monolayers were then visualized with a UV-light microscope (Carl Zeiss) after nuclei staining with Hoechst solution in the cell culture medium. Magnification x63. Bars, 10 µm.
**Figure 8****.** G SVCV-derived expressing cassette.

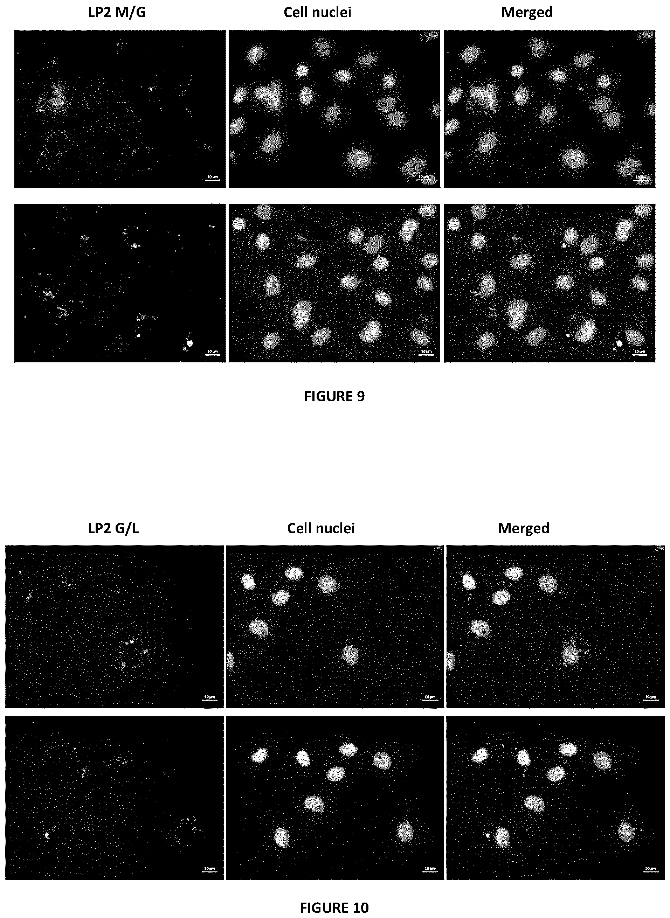

**Figure 9****.** Expression of LP2 protein in live rSVCV-LP2 M/G infected EPC cells. The cells were incubated at 25 °C for 24 h after infection. Membrane expression of LP2 antigen was visualized on live cells using anti-nodavirus polyclonal antibody kindly provided by Dr Anna Toffan. Cells were incubated with the anti-nodavirus in GMEM 2% for 45 min at room temperature (RT) and washed 3 times with GMEM 2%. Cells were then incubated with Alexa Fluor 594-conjugated anti-rabbit immunoglobulins (Invitrogen) for 45 min at RT. Cell monolayers were then visualized with a UV-light microscope (Carl Zeiss) after nuclei staining with Hoechst solution added in the cell culture medium. Magnification ×63. Bars, 10 µm.
**Figure 10****.** Expression of LP2 protein in live rSVCV-LP2 G/L infected EPC cells. The cells were incubated at 25 °C for 24 hours after infection. Membrane expression of LP2 antigen was visualized on live cells using anti-nodavirus polyclonal antibody kindly provided by Dr. Anna Toffan. Cells were incubated with the anti-nodavirus in GMEM 2% for 45 min at room temperature (RT) and washed 3 times with GMEM 2%. Cells were then incubated with Alexa Fluor 594-conjugated anti-rabbit immunoglobulins (Invitrogen) for 45 min at RT. Cell monolayers were then visualized with a UV-light microscope (Carl Zeiss) after nuclei staining with Hoechst solution added in the cell culture medium. Magnification ×63. Bars, 10 µm.

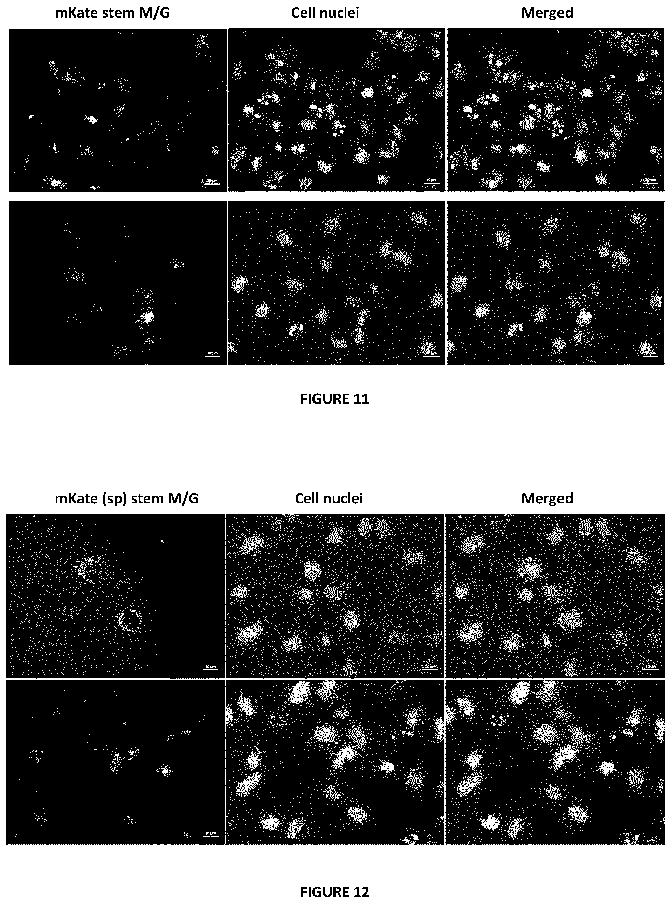

**Figure 11****.** Expression of mKate fluorescent protein in live rSVCV-mKate stem M/G infected EPC cells. The cells were incubated at 25 °C for 24 hours after infection. Cell monolayers were then visualized with a UV-light microscope (Carl Zeiss) after nuclei staining with Hoechst solution added in the cell culture medium. Magnification ×63. Bars, 10 µm.
**Figure 12****.** Expression of mKate fluorescent protein in live rSVCV-mKate sp stem M/G infected EPC cells. The cells were incubated at 25 °C for 24 hours after infection. Cell monolayers were then visualized with a UV-light microscope (Carl Zeiss) after nuclei staining with Hoechst solution added in the cell culture medium. Magnification ×63. Bars, 10 µm.

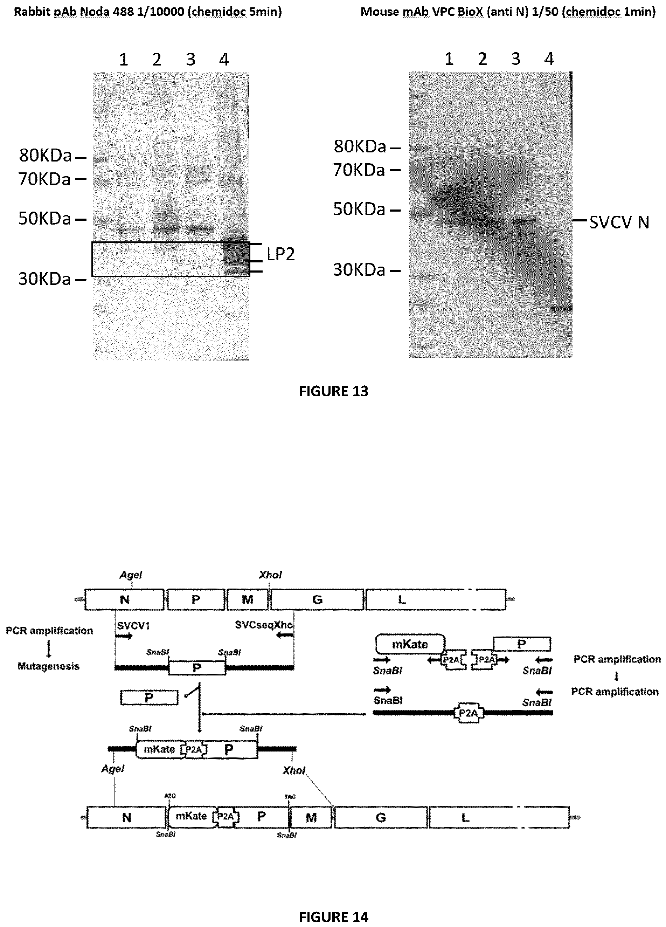

**Figure 13****.** Incorporation of LP2 protein in rSVCV-LP2 sp M/G viral particles. Proteins of sucrose gradient-purified viral particles were separated on a 12% polyacrylamide gel. A) Two micrograms of total viral proteins were denatured, loaded and migrated on SDS page gel. The gel was electrotransfered onto a PVDF membrane and Nodavirus LP2 (left panel) or SVCV N (right panel) were detected with a polyclonal antiboby (pAb) and a monoclonal antibody (mAb) directed against Nodavirus LP2 antigen and SVCV N, respectively. Lane 1: rSVCV LP2 M/G; lane 2: rSVCV LP2 sp M/G; lane 3: rSVCV WT; and lane 4: rVHSV LP2 (as a positive control).
**Figure 14****.** Construction of rSVCV mKate-P2A-P.

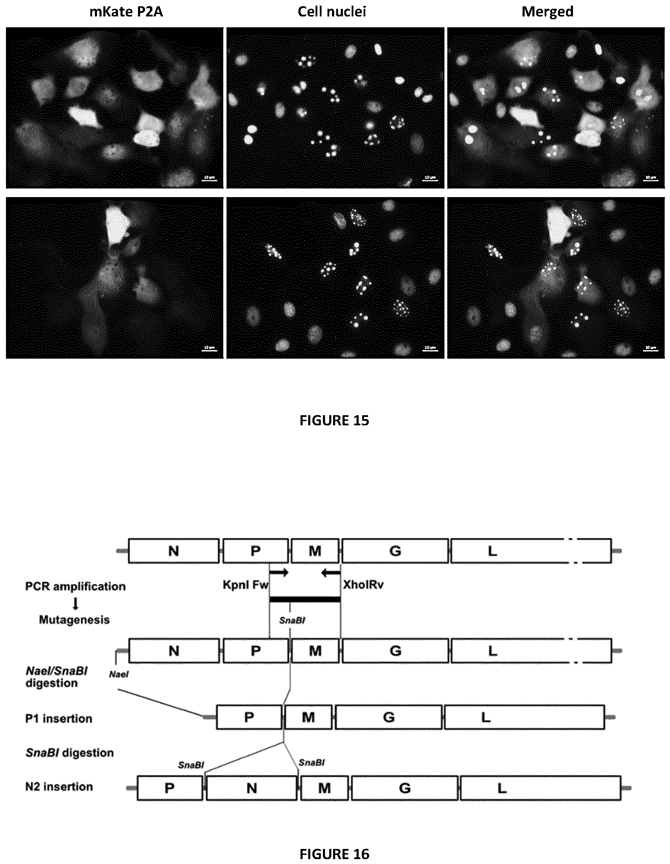

**Figure 15****.** Expression of mKate fluorescent protein in live rSVCV-mKate P2A infected EPC cells. The cells were incubated at 25 °C for 24 hours after infection. Cell monolayers were then visualized with a UV-light microscope (Carl Zeiss) after nuclei staining with Hoechst solution added in the cell culture medium. Magnification ×63. Bars, 10 µm.
**Figure 16****.** Construction of rSVCV-PN.

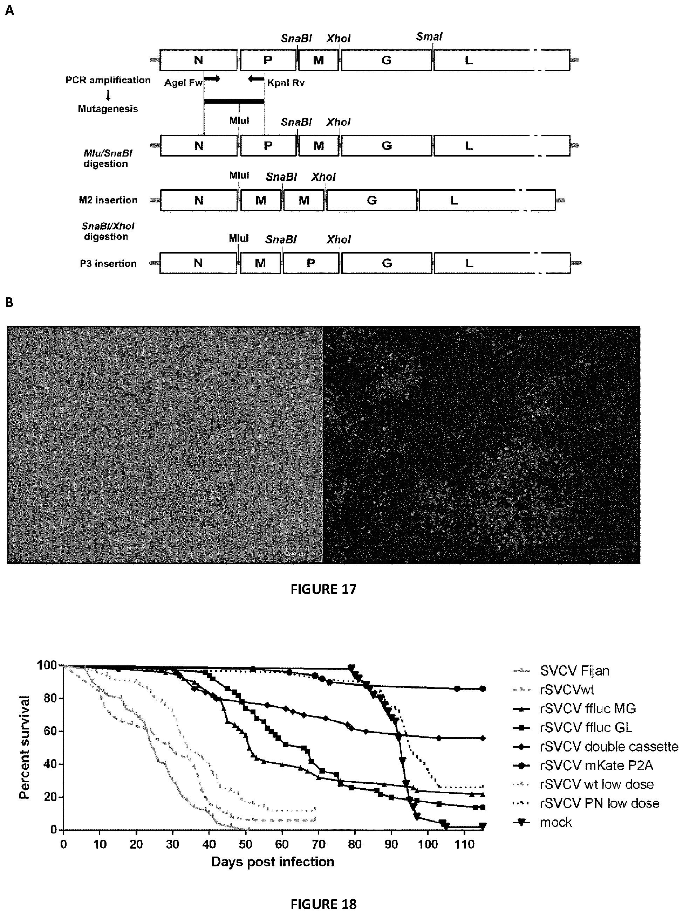

**Figure 17****.** Construction of rSVCV-MP and rSVCV-MP-mCherry P/G. A. Schematic construction of rSVCV-MP. The cassette expressing the mCherry fluorescent protein as described in Fig. 2, 3 and 4, was then introduced into the intergenic region between P and G genes, leading to rSVCV-MP-mCherry P/G. B. Expression of mCherry fluorescent protein in live rSVCV-MP-mCherry P/G infected EPC cells. The cells were incubated at 25 °C for 48 hours after infection. Bars, 100 µm.
**Figure 18****.** Experimental carp infection with rSVCVs and challenge.

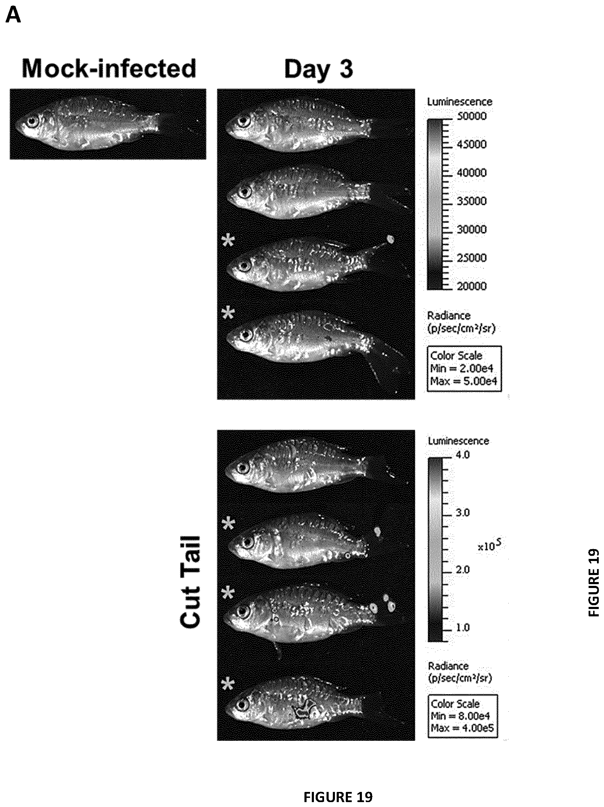

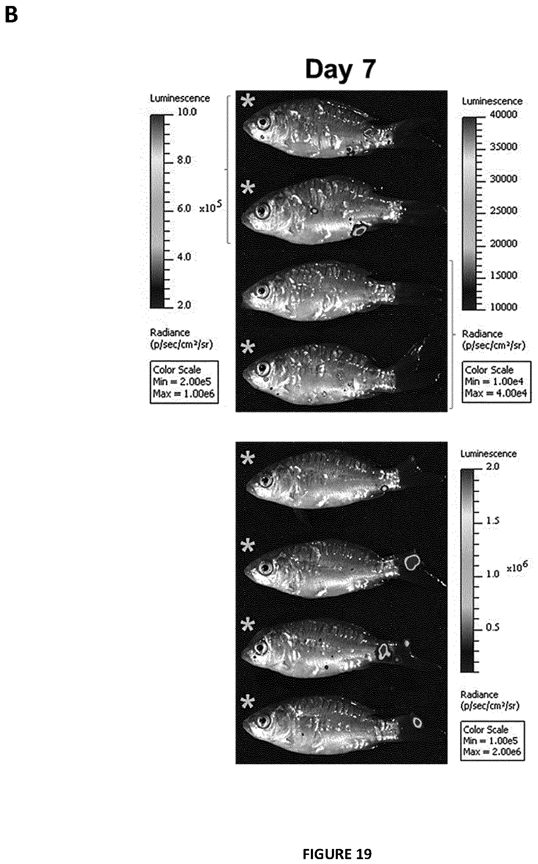

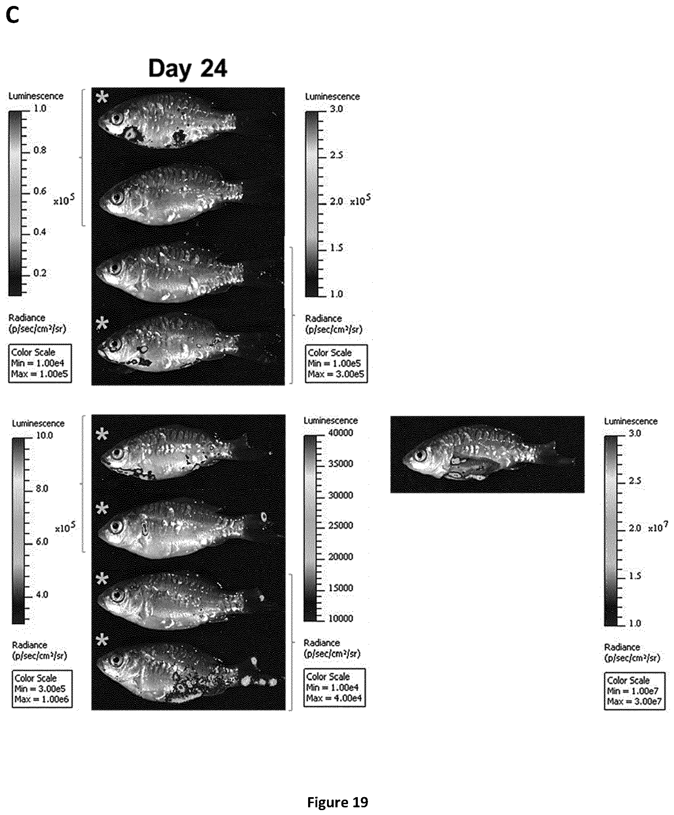

**Figure 19****.** Bioluminescence detection in fish of rSVCVakaLUC by bath immersion in akalumine substrate. Carp were divided into two groups with one group infected by immersion with rSVCVffLuc G/L or rSVCVakaLuc G/L as described above and the other group had their caudal fins cut prior to infection by immersion with rSVCVffLuc G/L or rSVCVakaLuc G/L (mean weight, 1.91 g). At 3-, 7-, and 24-days post infection, fish were randomly harvested and transferred in a small tank with water containing akalumine-HCI (Sigma) (40 mM at 1/10,000 dilution for 2 h at 10 °C). Two hours later, anesthetized fish were imaged using an IVIS Spectrum BL imaging system. Stars indicated positively-infected carp.

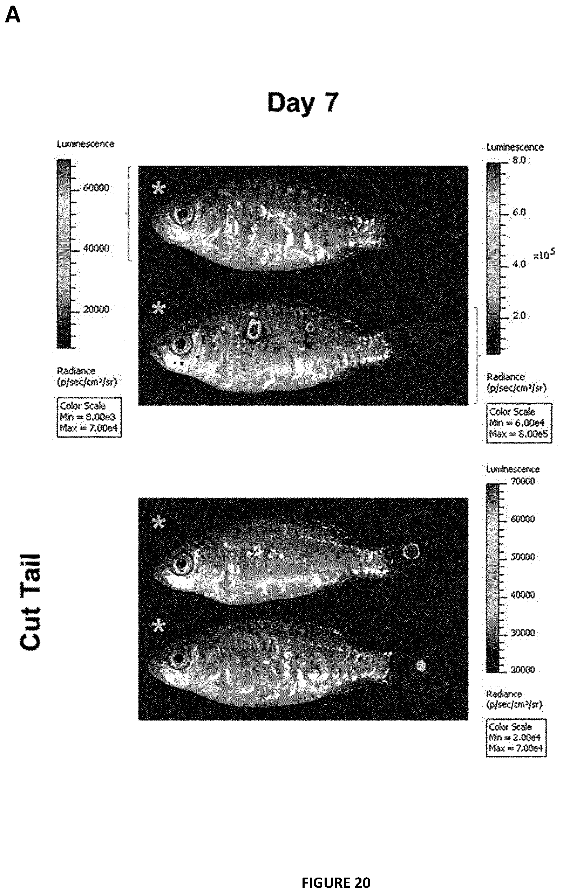

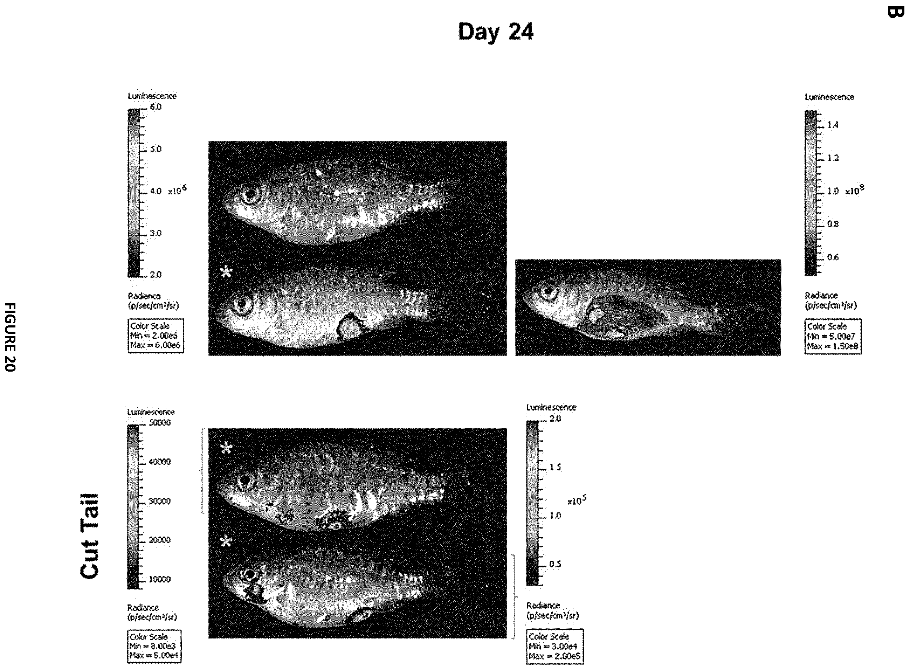

**Figure 20****.** Bioluminescence detection in fish of rSVCVakaLUC by injection of akalumine substrate. Carp were divided into two groups with one group infected by immersion with rSVCVffLuc G/L or rSVCVakaLuc G/L as described above and the other group had their caudal fins cut prior to infection by immersion with rSVCVffLuc G/L or rSVCVakaLuc G/L (mean weight, 1.91 g). At 7- and 24-days post infection, fish were randomly harvested and injected with 50 µL of akalumine-HCI at 1 mM. Five to ten minutes later anesthetized fish were imaged. Stars indicated positively-infected carp.

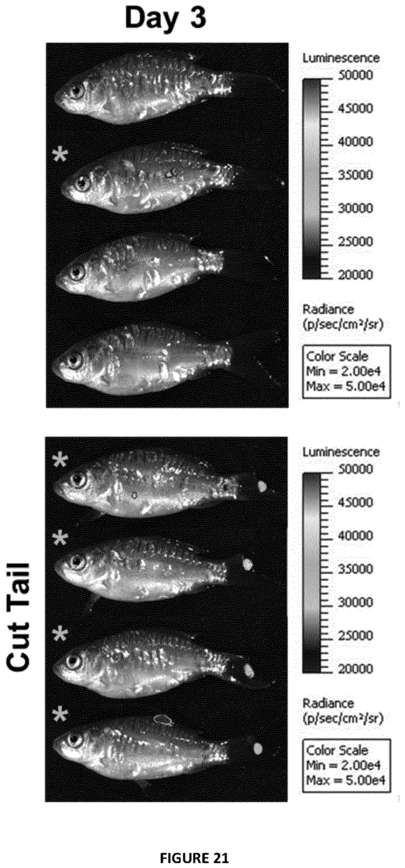

**Figure 21****.** Bioluminescence detection in fish of rSVCVffLUC by injection of D-luciferin substrate. Carp were divided into two groups with one group infected by immersion with rSVCVffLuc G/L as described above and the other group had their caudal fins cut prior to infection by immersion with rSVCVffLuc G/L (mean weight, 1.91 g). At 3 days post infection, fish were randomly harvested and injected with 50 µL of D-luciferin at 15 mg/mL. Five to ten minutes later anesthetized fish were imaged. Stars indicated positively infected carp.

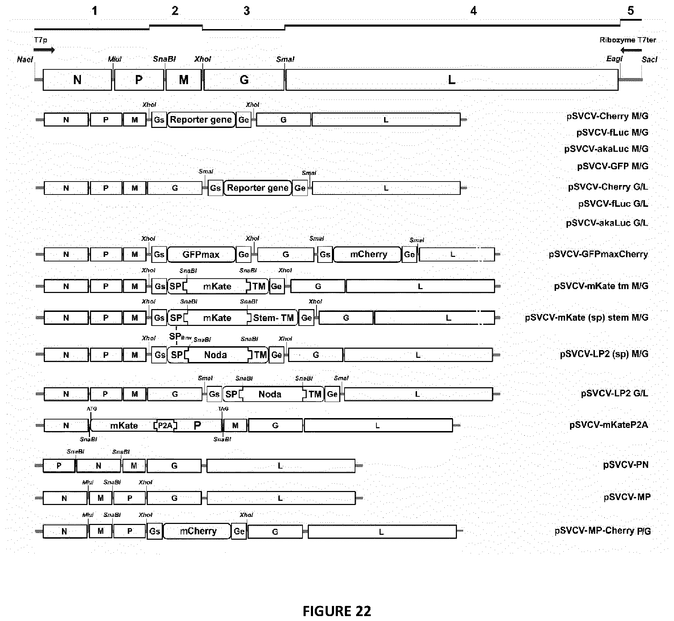

**Figure 22****.** Summary of rSVCV constructs.

### EXAMPLES

### Example 1. Construction of full-length antigenomic copies of the SVCV genome.

A full-length cDNA copy of the SVCV RNA genome was constructed by assembling five overlapping cDNA fragments generated through reverse transcription (RT)-PCR as depicted in **Figure 1****.**

The 5' end of fragment 1 (2,037 nt) consists of a *Nael* restriction enzyme (RE) site, a promoter sequence for the T7 RNA polymerase and three G residues to enhance promoter activity and fused to the first nucleotide of the SVCV antigenomic sequence. Fragment 1 covers the N gene and a large part of the P gene up to a naturally occurring *Kpnl* RE site. Fragment 2 (1,050 nt) and Fragment 3 (1,569 nt) introduce a *Xhol* (at positions 3064-3069 of the genome) and a *Smal* (at positions 4631-4636 of the genome) RE sites in the intergenic regions between the M and G genes and the G and - L genes, respectively. Fragment 4 (6,343 nt) comprises the complete L gene and includes an *Eagl* RE site at the 3' end (at position 10976-10981 of the genome). Finally, fragment 5 (213 nt) contains the *Eagl* RE site with the trailer sequence fused to the sequence of the hepatitis delta virus antigenomic ribozyme, followed by a T7 terminator sequence, and a *Sacl* RE site. All fragments were cloned into a pJET1.2 cloning vector, and the sequences were confirmed by DNA sequencing. Fragments 1 to 5 were sequentially cloned into a pBluescript SK- to obtain the complete SVCV antigenome construct, named pSVCV#2. This genome differs from the genome of the Fijan reference strain (GenBank # AJ318079) by the three additional RE sites described above and three nucleotide substitutions, two of which are synonymous mutations and are located in the N (T->G at position 1284) and G (A->G at position 3897) genes and one is a missense mutation in the G gene (A->G at position 4354) leading to threonine to alanine substitution.

### Example 2. Construction of a recombinant virus SVCV (rSVCV) expressing additional genes

### 2.1. Design of the SVCV expression cassette

The complete genomic sequences of 14 SVCV strains, that are available in GenBank under accession numbers AJ318079, EU177782, DQ097384, KJ513477, KR012466, KR012465, KR012467, KR012468, KU230365, KT321307, KY475636, MG663513, MG663514, and U181001.2, were aligned and the intergenic regions (N-P, P-M, M-G and G-L) were compared to obtain the minimal sequence DDDRTATGAAAAAAACTAACAGASATCATG (with D = G, A or T, R = G or A, and S = G or C, SEQ ID NO: 12); which comprises the untranslated region of SVCV gene containing the transcriptional termination/polyadenylation Gene End (GE) sequence, TATGAAAAAAA (SEQ ID NO: 13), and the transcription initiation Gene Start (GS) sequence, AACAGASATCATG (with S = G or C, SEQ ID NO: 14). These sequences will be used in the expression cassettes described for the insertion of an additional gene in SVCV genome.

### 2.2. Construction of rSVCV expressing additional genes

PCR fragments containing the coding region for green fluorescent protein (GFPmax and eGFP), red fluorescent protein mCherry or luciferase protein (firefly luciferase; fLUC and Akaluciferase; akaLUC) and flanked by the GS and GE transcription signals of SVCV **(****Figure 2****)** were obtained by PCR.

These final PCR products were digested with *Smal*-or *Xhol* and inserted into a *Xhol-* or *Smal-*linearized pSVCV construct, leading to pSVCV-mCherry M/G, pSVCV-mCherry G/L, pSVCV-eGFP M/G, pSVCV-fLUC M/G, pSVCV-fLUC G/L, pSVCV-akaLUC M/G, and pSVCV-akaLUC G/L **(****Figures 3-****6).** A pSVCV with two reporter genes was constructed by the insertion of GFPmax into the *Xhol* site and the mCherry gene into the *Smal* site leading to the pSVCV GFPmax-mCherry **(****Figure 7****).** For the insertion of the *Smal*-PCR products into the M-G intergenic region, the plasmid was treated by the Klenow fragment to obtain compatible blunt ends, after *Xhol* digestion.

### Example 3. Construction of rSVCV expressing proteins targeted to the plasma membrane of infected cells and to the viral envelope.

### 3.1. G SVCV-derived expressing cassette

A pcDNA3 plasmid containing the SVCV glycoprotein (G) gene was mutated to introduce two *SnaBI* RE sites by site-directed mutagenesis at the end of the signal peptide (SP; MSIISYIAFLLLIDSNLGI, SEQ ID NO: 15) and at the beginning of either the transmembrane domain (TM) or the stem region, using specific primers **(****Figure 8****).** Each G SnaB1-mutated ORF was amplified by PCR using primers that included the GE and GS transcription signals of SVCV **(****Figure 8****),** to obtain the SP/TM and SP/StemTM cassettes. The PCR products were integrated into the SVCV full-length genome in the intergenic positions M-G (*Xhol* digestion and klenow treatment) or G-L (*Smal* digestion). Finally, the additional gene expression cassettes were constructed by replacement of SVCV G ectodomain-stem or ectodomain with the gene of interest (betanodavirus antigen LP2 or mKate red fluorescent protein).

The betanodavirus antigen (LP2), which is composed of two copies of the linker and protruding domains of the capsid protein, was amplified by PCR from the pBS160R2 plasmid (Souto et al., J Gen Virol. 96:1287-1296, 2015) with primers *SnaBI* LinkP2 Fw/Rv, and inserted into the *SnaBI-*digested pSVCV-SP/TM M/G or G/L and leading to pSVCV-LP2 M/G and pSVCV-LP2 G/L **(****Figures 9, 10****).** The mKate fluorescent protein ORF was amplified with the primers KateFw/Rv and inserted in position M/G using the SP/TM cassette, leading to pSVCV-mKate M/G, and to improve the membrane insertion efficacy of the protein, using the SP/StemTM cassette leading to pSVCV-mKate stem M/G **(****Figure 11****).**

### 3.2. Chimeric expressing cassette

Chimeric SVCV cassettes were generated to enhance protein expression and incorporation into the SVCV virion envelope. The SP derived from the *Infectious hematopoietic necrosis virus* (IHNV) glycoprotein (G) gene and the stem-TM/TM from SVCV were genetically fused to the amino and carboxy termini of the mKate and LP2 genes. The IHNV SP sequence ATGGACACCACGATCACCACTCCGCTCATTCTCATTCTGATCACCTGCGGAGCA (SEQ ID NO: 16) was incorporated by PCR using the primers SP IHN_mKate or SP IHN_LP2 **(****Figure 8****),** with the reverse primer Gsvcv 3'lnt. The resulting Smal-PCR products were cloned into the M-G intergenic region of pSVCV#2 (*Xhol* digestion and klenow treatment), leading to pSVCV mKate sp stem M/G and pSVCV LP2 sp M/G **(****Figures 12****,** **13****).** The incorporation efficiency of LP2 antigen at the surface of recombinant SVCV on sucrose-purified viral particles was verified by Western-blot assay. **Figure 13** shows that rSVCV LP2 sp M/G expresses and incorporates LP2 antigen.

### 3.3. G SVCV-derived expressing cassette, 2^{nd} version

The previous SVCV G SP/TM cassette in position M/G has been modified by the addition of two additional amino acids in order to extend the SP sequence and increase the membrane expression of the gene of interest leading to the following SP sequence: MSIISYIAFLLLIDSNLGIPI (SEQ ID NO: 9). The two additional amino acids (proline and isoleucine; PI) were introduced by site-directed mutagenesis in both pSVCV-mKate M/G and pSVCV-mKate stem M/G constructs.

### Example 4. Construction of rSVCV mKate-P2A-P

RE digestion fragment by *Agel*/*Xhol* of pSVCV#2 (2,318 nt; **Figure 14****),** which included the SVCV phosphoprotein (P) gene, was amplified by PCR using the primers SVC1/ SVCseqXho from pSVCV#2 and inserted into a pJET1.2 cloning vector. Two *SnaBI* restriction sites were inserted at the beginning and at the end of the P ORF by mutagenesis using the primers MutATGSnaB and MutTAGSnaB leading to the pJet-SVCV Age/XhoMut.

The mKate ORF and the P ORF were amplified by PCR using the primers mKateSnaFw/P2A Rv and PsvcvP2A Fw/PsvcvSnaRv respectively. One primer of each pair was designed to include an overlapping sequence of a P2A sequence derived from porcine teschovirus, which is fused to the 3' end of the mKate gene, removing the stop codon, and to the 5' end of the P gene.

The P2A sequence can induce ribosomal skipping during translation, leading to the expression of two independent proteins from a unique messenger RNA (mRNA). Both PCR products were used as template in a new PCR with the primers mKateSnaFw/PsvcvSnaRv to create the mKate- P2a-P ORF, as depicted in **Figure 14****.** This PCR product was first cloned into the pJET-SVCV Age/XhoMut, replacing the P gene by *SnaBI* digestion, and then inserted into the SVCV genome using the *Agel*/*Xhol* sites, leading to pSVCV-mKateP2A **(****Figure 15****).**

### Example 5. Construction of rSVCV with modified gene order: rSVCV-PN

A fragment spanning the region from the *Kpnl* RE site (P gene) to the *Xhol* RE site (M-G intergenic region) of 1,060 bp was amplified from pSVCV#2 and inserted into a pJET1.2 cloning vector to incorporate by mutagenesis a *SnaBI* site downstream of the P gene **(****Figure 16****).** Following mutagenesis, a *Kpnl*/*Xhol-mutSnaBI* fragment was excised from the pJET vector and inserted back into the *Kpnl*/*Xhol-digested* pSVCV#2, leading to the pSVCV-mut*SnaB*. The P and N SVCV ORFs were amplified by PCR using the primer pairs *Nael* P1 Fw/*SnaB* P1 Rv and *SnaB* N2 Fw/Rv, respectively using pSVCV#2 as template. The P1 fragment includes a *Nael* RE site at 5', followed by the T7 promoter and the SVCV leader sequence fused to the ATG of the P gene, and a *SnaBI* RE site at the 3' end. The fragment N2 is flanked by *SnaBI* RE sites and contains the original SVCV N-P intergenic region fused to the ATG of the N gene. Fragment P1 was digested by *Nael*/*SnaBI* and cloned into the *Nael*/*SnaBI*-pSVCV-mutSnaB. Finally, Fragment N2 was digested by *SnaBI* and cloned into the *SnaBI*-pSVCV-P1, leading to the final construct pSVCV-PN. This construction contains nucleotide substitutions in the non-coding region at positions 1332-1334 and at positions 2341-2344 of the genome; that correspond to the *SnaBI* RE sites introduced downstream of the N and P genes, respectively.

### Example 6. Construction of rSVCV with modified gene order: rSVCV-MP

A fragment spanning the region from the *Agel* RE site (N gene) to the *Kpnl* RE site (P gene) of 1,241 bp was amplified from pSVCV#2 and inserted into a pJET1.2 cloning vector to incorporate by mutagenesis a *MluI* site downstream of the N gene **(****Figure 17A****).** Following mutagenesis, a *Agel*/*Kpnl*-mutMlu fragment was excised from the pJET vector and inserted back into the *Kpnl*/*Xhol-*digested pSVCV-mutSnaB, leading to the pSVCV-mutMlu&SnaB. The P and M SVCV ORFs were amplified by PCR using the primers pairs SnaB P Fw/Xho P Rv and Mlu M Fw/SnaB M Rv, respectively using pSVCV#2 as template. The P3 fragment is flanked by *snaBI* and *Xhol* RE sites and contains the original SVCV P-M intergenic region fused to the ATG of the P gene. The fragment M2 is flanked by *MluI* and *SnaBI* RE sites and contains the original SVCV N-P intergenic region fused to the ATG of the M gene. Fragment P was digested by *SnaBI*/*Xhol* and cloned into the *SnaBI*/*Xhol-*pSVCV-mutMlu&SnaB. Finally, Fragment M was digested by *MluI*/*SnaBI* and cloned into the *MluI*/*SnaBI-*pSVCV-P3, leading to the final construct pSVCV-MP. This construction contains nucleotide substitutions in the non-coding region at positions 1373-1376 and at positions 2341-2344 of the genome that correspond to the *MluI* and *SnaBI* RE sites introduced downstream of the N and P genes, respectively. Finally, the cassette expressing the mCherry fluorescent protein as described in Figures 2 3 and 4, was introduced into the intergenic region between P and G genes using the unique *Xhol* restriction site, leading to rSVCV-MP-mCherry P/G (**Figure 17B**).

### Example 7. Production of recombinant SVCV viruses in transfected BSRT7/5 cells by a DNA-based reverse genetics system

### 7.1. Cell culture

BSRT7/5 cells (Buchholz et al., J. Virol., 73:251-259, 1999) are a cell line derived from BHK-21 cells (Baby hamster kidney cells) that constitutively express the T7 RNA polymerase of the T7 bacteriophage. BSRT7/5 cells are grown in 75 cm² flasks and subcultured once or twice per week at a ratio of 1:6. The cells were incubated in a 5% CO₂ humidified atmosphere at 37 °C and were maintained in Dulbecco's Modified Eagle medium (DMEM, Eurobio) supplemented with 2 mM L-glutamine (PAA), 10% fetal bovine serum (FBS, Eurobio) and the antibiotics penicillin (100 U/mL) and Streptomycin (0.1 mg/mL). Cells were incubated in culture medium containing Geneticin (G418, Gibco, 1 mg/mL, final concentration) every two passages.

Recombinant SVCV viruses (rSVCV) were propagated using monolayer cultures of Epithelioma papulosum cyprinid (EPC) cells maintained at 25 °C in Glasgow's modified Eagle's medium (GMEM) containing 25 mM 4-(2-hydroxyethyl)-1-piperazineethanesulfonic acid (HEPES, Biosera) supplemented with 2% fetal bovine serum (FBS, Capricorn Scientific), 1% tryptose phosphate broth (Sigma), 2 mM L-glutamine (Eurobio) and penicillin (100 U/mL)/streptomycin (100µg/mL) (Biovalley).

### 7.2. Production of recombinant viruses (rSVCV)

rSVCV were generated by transfecting BSRT7/5 cells with a mixture of four plasmids by lipofection with the transfection reagent Lipofectamine 2000 (Invitrogen). For virus recovery, the day before transfection, 1 × 10⁶ cells/well were seeded in wells of six-well plates so as to reach 90% confluence the next day. The day of transfection, cells were maintained in DMEM medium without antibiotics and serum. Cells were transfected with 5 µg of pSVCV plasmid DNA (construction of recombinant antigenomic cDNA, **Figure 1****)** and the helper plasmids: pSVCV-N (2 µg), pSVCV-P (2 µg) and pSVCV-L (1 µg), which encode the nucleoprotein N, the phosphoprotein P, and the RNA-dependent RNA polymerase L, respectively (cloned in pCITE plasmids). First, plasmids and Lipofectamine (1:3 ratio) were diluted separately in 150 µL of Opti-MEM^{®} Medium (Gibco) and afterwards diluted DNA was added to Lipofectamine. The DNA-lipid complex was incubated for 5 minutes at room temperature and then was added to cells drop-wise. After transfection, the cells were incubated for 6 h to 12 h at 37 °C, the transfection mix was then removed and the cells were shifted to 25 °C with GMEM 2% FBS for 7 days. After that, cells were scraped and mixed with the supernatant by scratching wells (the recovered virus at this stage corresponds to passage 0, or P0), and were used to infect a monolayer of fresh EPC cells seeded in 24-well plates and incubated at 25 °C (dilution 1:10 in GMEM medium). After 1-4 days, when the virus was produced, the supernatant from this culture (supernatant of passage 1, or P1) was collected and used to infect fresh fish cells (dilution 1:100 in GMEM medium). Supernatant of P2 was titrated and stored at -80 °C.

### 8. Experimental fish infection.

Virus-free juvenile carp (50 fish per groups; mean weight, 0.81 g) were infected by immersion in tanks filled with 3 liters of freshwater with recombinant rSVCV viruses (final titer, 5 × 10⁴ PFU/mL and 10⁴ PFU/mL for low dose group) for 2 h at 10 °C. Tanks were then filled up to 30 L of freshwater. Control fish were mock infected with cell culture medium under the same conditions. Mortalities were recorded every day over a period of 115 days. For rSVCV variants, surviving carp were challenged by immersion at day 69 with the virulent SVCV Fijan strain as described above (final titer, 5 × 10⁴ PFU/mL) and mortality was recorded up to day 115.

**Figure 18** shows the cumulative percentage of mortality registered in each tank. Mortality started as soon as 6 to 10 days post infection and reached 94% to 100% of cumulative mortality at roughly 50 days post infection for each virus. The kinetics of mortality for the lower SVCV dose (10⁴ PFU/mL) follows a similar curve to that of the higher dose except for a delay of a few days and reached 88% of cumulative mortality at day 56 post infection. During the acute phase, moribund fish displayed typical petechial hemorrhages of the skin, irrespective of the virus inoculum used. No mortality was observed in the mock-infected tank.

Taken together with the data from *in vitro* infections and titers, this confirmed that rSVCVwt was fully competent for growth *in vitro* and was as pathogenic as SVCV Fijan *in vivo,* indicating that the SVCV sequence used in the present reverse genetics system is functional and appears to encode a wild-type virus.

Recombinant viruses containing one or two additional expression cassettes were attenuated compared to wild-type viruses (SVCV Fijan and rSVCVwt). Mortality induced by rSVCV double cassette, rSVCV ffLUC GL and rSVCV ffLUC MG started at 15 to 30 days post infection and reached 30, 58% and 64% of cumulative mortality, respectively, at day 69 post infection. rSVCVmKateP2A and rSVCV-PN were highly attenuated with only 6% and 4% of induced cumulative mortality, respectively.

Surviving fish from the initial infection trial were challenged with SVCV Fijan at days 69 post primary infection. The mock-infected fish control groups were highly susceptible to SVCV Fijan challenge with 98% of cumulative mortality 36 days post challenge. In the group initially infected with rSVCV-PN, a high mortality rate was observed (70% of cumulative mortality 34 days post challenge), meaning that the initial infection by rSVCV-PN did not induce a strong immune protection. In contrast, only 8% of cumulative mortality was recorded for the group initially infected with rSVCVmKateP2A at day 115. The calculated relative percent of survival for this group is very high and reaches 86%, meaning that this recombinant virus is highly attenuated but still highly immunogenic and protective **(Table 1).**

**Table 1. Summary of rSVCV-infected fish from the experiment depicted in Figure 18. ^{a}Cumulative percent of mortality at day 69 postimmunization. ^{b} SVCV challenge was performed at day 69 postimmunization and ended at day 115. ^{c} Relative percent survival (RPS) = 1 - (percent mortality in group/percent mortality in control) × 100 (Amend DF. 1981. Potency testing of fish vaccines. Dev Biol Stand 49:447-454.). ^{d} Group of 50 fish immunized with virus-free culture medium and challenged with SVCV at day 69 postimmunization.**

| **Virus** | **% cumulative mortality** | | **RPS^{c}** |
|---|---|---|---|
| | **Immunization^{a}** | **Challenge^{b}** | |
| rSVCVffLuc M/G | 64 | 14 | 20 |
| rSVCVffLuc G/L | 58 | 28 | 12 |
| rSVCVdouble cassette | 30 | 14 | 55 |
| **rSVCVmKateP2A** | **6** | **8** | **86** |
| rSVCV-PN | 4 | 70 | 24 |
| Control^{d} | 0 | 98 | - |

### 9. Experimental fish infection and bioluminescence imaging.

For *in vivo* bioluminescence imaging, carp were divided into two groups with one group infected by immersion with rSVCVffLuc G/L or rSVCVakaLuc G/L as described above and the other group had their caudal fins cut prior to infection by immersion with rSVCVffLuc G/L or rSVCVakaLuc G/L (mean weight, 1.91 g). At 3-, 7-, and 24-days post infection, 12-16 fish were randomly transferred in a small tank with water containing akalumine-HCI (Sigma) (40 mM at 1/10,000 dilution). Two hours later, anesthetized fish were subjected to imaging using an IVIS Spectrum BL imaging system (PerkinElmer). Living Image software (version 4.7.3, PerkinElmer) was used to acquire both bioluminescent and photographic images. Some of the fish were injected with 50 µL of either 15 mg/mL D-luciferin (PerkinElmer) or 1 mM akalumine-HCl. Five to ten minutes later anesthetized fish were imaged.

*In vivo* bioluminescence imaging results are shown in **Figures 19-21****.**

### 9. Summary of rSVCV constructs

A summary of rSVCV constructs is given in **Figure 22****.**

## Claims

1. A recombinant virus of the genus *Sprivivirus* comprising in its genome at least one exogenous genetic sequence.

2. The recombinant virus according to claim 1 comprising in its genome at least one exogenous genetic sequence encoding a heterologous gene, in addition to the endogenous viral genes encoding nucleoprotein (N), phosphoprotein (P), matrix protein (M), glycoprotein (G) and polymerase (L).

3. The recombinant virus according to claim 1 comprising in its genome at least one exogenous genetic sequence encoding a heterologous gene, in substitution of an endogenous viral gene encoding nucleoprotein (N), phosphoprotein (P), matrix protein (M) or glycoprotein (G).

4. The recombinant virus according to any one of claims 1 to 3, wherein the exogenous genetic sequence comprises or consists of an expression cassette encoding a peptide or protein of interest, which comprises, from 3' end to 5' end:
- a transcription initiation Gene Start (GS) sequence chosen from the group consisting in SEQ ID NO: 1 (UUGUCUCUAGUAC) and SEQ ID NO: 2 (UUGUCUGUAGUAC),
- a sequence encoding the protein of interest, and
- a transcriptional termination/polyadenylation Gene End (GE) sequence consisting in SEQ ID NO: 3 (AUACUUUUUUU),
said expression cassette being inserted in a non-coding region of the genome.

5. The recombinant virus according to any one of claims 1 to 3, wherein the exogenous genetic sequence comprises:
- a 2A-like sequence, which encodes a peptide that induces ribosomal skipping during translation, and
- a sequence encoding a protein of interest,
said exogenous genetic sequence being fused to the translated region of an endogenous viral gene encoding N, P, M, G or L, and the 2A-like sequence being located between the sequence encoding the protein of interest and the translated region of the endogenous viral gene encoding N, P, M, G or L.

6. The recombinant virus according to claim 5, wherein the 2A-like sequence encodes a peptide selected from the group consisting in P2A (ATNFSLLKQAGDVEENPGP, SEQ ID NO: 5), T2A (EGRGSLLTCGDVEENPGP, SEQ ID NO: 6), E2A (QCTNYALLKLAGDVESNPGP, SEQ ID NO: 7) and F2A (VKQTLNFDLLKLAGDVESNPGP, SEQ ID NO: 8).

7. The recombinant virus according to any one of claims 1 to 2 and 4 to 6, wherein the endogenous viral genes encoding nucleoprotein N, P, M, G and L are not in the following arrangement 3' N-P-M-G-L 5'.

8. The recombinant virus according to any one of claims 1 to 7, said recombinant virus expressing a protein of interest flanked at its N-terminus by the signal peptide sequence of G protein and at its C-terminus by the sequence of the transmembrane domain of G protein or the sequence of the membrane-proximal stem region and the transmembrane domain of G-protein.

9. The recombinant virus according to any one of claims 1 to 8, said virus being chosen from the group comprising *Carp sprivivirus* and *Pike sprivivirus* species, preferably from the group comprising spring viraemia of carp virus (SVCV), pike fry rhabdovirus (PFRV), grass carp rhabdovirus (GCRV) and tench rhabdovirus (TRV).

10. A cDNA molecule of the antigenome of a recombinant virus as defined in any one of claims 1 to 9.

11. Method for producing a recombinant virus as defined in any one of claims 1 to 9, said method comprising a step of cultivating some hamster kidney cells expressing T7 RNA polymerase that have been transfected with a cDNA molecule of the antigenome of the recombinant virus and at least one helper plasmid encoding N, P and L.

12. A recombinant virus as defined in any one of claims 1 to 9 for use as a vaccine.

13. The recombinant virus for its use as defined above, wherein said vaccine is intended to be administered to an animal, preferably a fish, a bird or a mammal, more preferably a fish, even more preferably a fish chosen from the group comprising common carp (*Cyprinus carpio carpio*)*,* koi carp (*Cyprinus carpio koi*)*,* crucian carp (*Carassius carassius*), silver carp (*Hypophthalmichthys molitrix*)*,* bighead carp (*Aristichthys nobilis*)*,* grass carp (white amur) (*Ctenopharyngodon idella*), goldfish (*Carassius auratus*), orfe (*Leuciscus idus*)*,* tench (*Tinca tinea*), bream (Abramis brama), European catfish or wels (*Silurus glanis*), pike (*Esox lucius),* Nile tilapia (*Sarotherodon niloticus*)*,* Siberian sturgeon (*Acipenser baerii*)*,* Zebrafish (*Danio rerio*)*,* Golden shiner (*Notemigonus crysoleucas*)*,* Fathead minnow (*Pimephales promelas*), Caspian white fish (*Rutilus kutum*) and Roach (*Rutilus rutilus*)*.*

14. A non-therapeutic method for producing antibodies directed against an antigenic protein of interest, said method comprising the immunization of a non-human animal with a recombinant virus as defined in any one of claims 1 to 9, said recombinant virus expressing the antigenic protein of interest.
